# EUROPEAN PATENT SPECIFICATION

(11) **EP 2 590 669 B1**
(45) Date of publication and mention of the grant of the patent: **31.08.2016**
(21) Application number: 11734062.0
(22) Date of filing: 11.07.2011
(51) Int. Cl.: A61K 38/40, A61P 7/02

(54) **TREATMENT OF A DISEASE ASSOCIATED WITH RETINAL DEGENERATIVE DISORDER**
BEHANDLUNG VON KRANKHEITEN IM ZUSAMMENHANG MIT DEGENERATIVEN NETZHAUTSTÖRUNGEN
TRAITEMENT D'UNE MALADIE ASSOCIÉE À UN TROUBLE DÉGÉNÉRATIF DE LA RÉTINE

(30) Priority: 09.07.2010 EP 10305765
(43) Date of publication of application: 15.05.2013
(73) Proprietor: INSERM (Institut National de la Santé et de la Recherche Médicale), 75013 Paris (FR); Université Paris Descartes, 75006 Paris (FR); Assistance Publique - Hôpitaux de Paris, 75004 Paris (FR)
(72) Inventor: COURTOIS, Yves, F-75270 Paris Cedex 06 (FR); JEANNY, Jean-Claude, F-75270 Paris Cedex 06 (FR); PICARD, Emilie, F-75270 Paris Cedex06 (FR); BEHAR-COHEN, Francine, F-75270 Paris Cedex06 (FR)
(74) Representative: Hirsch, Denise Marie
(86) International application number: PCT/EP2011/061784
(87) International publication number: WO 2012/004416

(56) References cited:
- WO-A2-2005/112911
- US-A1- 2007 149 440
- PICARD EMILIE ET AL: "The protective role of transferrin in Muller glial cells after iron-induced toxicity", MOLECULAR VISION, MOLECULAR VISION, SN, ATLANTA , vol. 14, no. 111 20 May 2008 (2008-05-20), pages 928-941, XP009138623, ISSN: 1090-0535 Retrieved from the Internet: URL:http://www.molvis.org/molvis/v14/a111
- DELEON EFRAT ET AL: "Alteration in Iron Metabolism during Retinal Degeneration in rd10 Mouse", INVESTIGATIVE OPHTHALMOLOGY & VISUAL SCIENCE, ASSOCIATION FOR RESEARCH IN VISION AND OPHTHALMOLOGY, US LNKD- DOI:10.1167/IOVS.08-1856, vol. 50, no. 3, 1 March 2009 (2009-03-01), pages 1360-1365, XP009138619, ISSN: 0146-0404 [retrieved on 2008-11-07]
- CHOWERS ITAY ET AL: "The iron carrier transferrin is upregulated in retinas from patients with age-related macular degeneration", INVESTIGATIVE OPHTHALMOLOGY & VISUAL SCIENCE, ASSOCIATION FOR RESEARCH IN VISION AND OPHTHALMOLOGY, US, vol. 47, no. 5, 1 May 2006 (2006-05-01), pages 2135-2140, XP009138620, ISSN: 0146-0404
- CHEN H ET AL: "Changes in iron-regulatory proteins in the aged rodent neural retina", NEUROBIOLOGY OF AGING, TARRYTOWN, NY, US LNKD- DOI:10.1016/J.NEUROBIOLAGING.2008.01.002, vol. 30, no. 11, 1 November 2009 (2009-11-01), pages 1865-1876, XP026601860, ISSN: 0197-4580 [retrieved on 2008-03-04]
- HE ET AL: "Iron homeostasis and toxicity in retinal degeneration", PROGRESS IN RETINAL AND EYE RESEARCH, OXFORD, GB LNKD- DOI:10.1016/J.PRETEYERES.2007.07.004, vol. 26, no. 6, 24 October 2007 (2007-10-24), pages 649-673, XP022313836, ISSN: 1350-9462
- DATABASE MEDLINE [Online] US NATIONAL LIBRARY OF MEDICINE (NLM), BETHESDA, MD, US; June 2004 (2004-06), LÉCUREUIL C ET AL: "Transgenic mice as a model to study the regulation of human transferrin expression in Sertoli cells.", XP009138878, Database accession no. NLM15105395 & HUMAN REPRODUCTION (OXFORD, ENGLAND) JUN 2004 LNKD- PUBMED:15105395, vol. 19, no. 6, June 2004 (2004-06), pages 1300-1307, ISSN: 0268-1161
- PICARD EMILIE ET AL: "Overexpressed or intraperitoneally injected human transferrin prevents photoreceptor degeneration in rd10 mice.", MOLECULAR VISION 2010 LNKD- PUBMED:21179240, vol. 16, 2010, pages 2612-2625, XP009153667, ISSN: 1090-0535

## Description

### FIELD OF THE INVENTION:

The present invention relates to human Transferrin or an active fragment thereof for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury

### BACKGROUND OF THE INVENTION:

All cells require iron for survival and as a cofactor of a variety of enzymes. However, it is also highly toxic due to its ability to generate free radicals via the Fenton reaction. Iron retinal homeostasis was regulated by proteins involved in iron import (transferrin (Tf), transferrin receptor (TfR)), storage (ferritin (Ft)) and export (ceruloplasmin (Cp), ferroportin (Fp), hephaestin (Hp)), thus preventing deleterious consequences of either iron overload or deficiency. The study of the iron metabolism in rodent retina has been partially elucidated by the localization of iron in the different retinal layers but also by the determination of the various enzymes involved in its homeostasis. Transferrin is mainly expressed in the retinal pigmented epithelium (RPE) and in the photoreceptors (PR). TfR and Ft are present in all outer retinal layers. Cp, Hp and Hep have also been identified in retina.

Diseases such as aceruloplasminemia and age-related macular degeneration (AMD) are associated with increased intraocular iron level, which contributes to oxidative injury and subsequent retinal degeneration. Iron was found in RPE, Bruch's membrane and PR layer from AMD patients. The macula of the eye from patient with geographic atrophy also had elevated levels of Tf, Ft and Fp in the PR and along the internal limiting membrane.

The inventors studied the iron accumulation during the course of retinal degeneration in rd10 retina by the Proton-Induced X-Ray Emission technique. Surprisingly, they show that human Transferrin (hTf) injections may protect on retinal degeneration in mice.

### SUMMARY OF THE INVENTION:

The invention is based on the discovery that hTf, by binding to iron, may protect the retina from degeneration due to iron accumulation.

Thus, the invention relates to human Transferrin or an active fragment thereof for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury

A further object of the invention relates to a pharmaceutical composition for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury comprising a therapeutically effective amount of human transferrin or an active fragment thereof according to claims 1 or 2, or an nucleic acid according to claim 3, or a plasmid according to claim 4, or an expression vector according to claim 5 along with at least one pharmaceutically acceptable excipient.

### DETAILED DESCRIPTION OF THE INVENTION:

### Protein and uses thereof:

A first object of the invention relates to the human Transferrin or an active fragment thereof for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury.

As used herein, the term "Transferrin" or "Tf" denotes a blood plasma protein for iron delivery. Transferrin is a glycoprotein that binds iron very tightly but reversibly. Although iron bound to transferrin is less than 0.1% (4 mg) of the total body iron, it is the most important iron pool, with the highest rate of turnover (25 mg/24 h). Transferrin has a molecular weight of around 80 kiloDaltons and contains 2 specific high-affinity Fe(III) binding sites. The affinity of transferrin for Fe(III) is extremely high (10.23 M-1 at pH 7.4) but decreases progressively with decreasing pH below neutrality. A sequence for human Transferrin gene is deposited in the database NCBI under accession number NM_001063.

As used herein, the term "an active fragment" denotes a fragment of a protein that retains the activity of the complete protein or has been modified to have increased binding property as compared to the full length native "Transferrin". For example, an active fragment of the Transferrin denotes a fragment of the protein which conserves the capacity to binding the iron.

As used herein, the terms "treating" or "treatment", denotes reversing, alleviating, inhibiting the progress of, or preventing the disorder or condition to which such term applies, or one or more symptoms of such a disorder or condition.

According to the invention, the term "patient" or "individual" to be treated is intended for a human or non-human mammal (such as a rodent (mouse, rat), a feline, a canine, or a primate) affected or likely to be affected with vision defects. Preferably, the subject is a human.

The capacity to binding the iron of the hTf or the active fragment thereof will become evident to the skilled person by implementing a simple test to evaluate the binding of iron of said proteins. For instance iron is readily removed from transferrin by incubation in a buffer containing 1mMNTA, 1mM EDTA, 0.5 M sodium acetate pH 4.9 The apoprotein is concentrated to a minimum volume on a centricon 10 (amicon), then diluted and reconcentrated twice with water and twice with 0.I NKCl. The apoprotein has a tendency to precipitate in pure water but redissolves in 0.1NMKCL.

In a preferred embodiment, said active fragment of hTf comprises at least 75% identity over said hTf, even more preferably at least 80%, at least 85%, at least 90%, at least 95%, at least 97%, at least 99%.

Typically said hTf or active fragment thereof may be used in combination with a compound against a disease associated with retinal degenerative disorder.

hTf or active fragment thereof may be produced by any technique known per se in the art, such as, without limitation, any chemical, biological, genetic or enzymatic technique, either alone or in combination(s).

Knowing the amino acid sequence of the desired sequence, one skilled in the art can readily produce a relevant part of the hTf or the active fragment thereof, by standard techniques for production of proteins. For instance, they can be synthesized using well-known solid phase method, preferably using a commercially available protein synthesis apparatus (such as that made by Applied Biosystems, Foster City, California) and following the manufacturer's instructions.

Alternatively, hTf or active fragment thereof can be synthesized by recombinant DNA techniques as is now well-known in the art. For example, these fragments can be obtained as DNA expression products after incorporation of DNA sequences encoding the desired polypeptide into expression vectors and introduction of such vectors into suitable eukaryotic or prokaryotic hosts that will express the desired protein or fragment of the protein, from which they can be later using well-known techniques.

hTf or active fragment thereof can be used in a vector, such as a membrane or lipid vesicle (e.g. a liposome).

In specific embodiments, it is contemplated that the hTf or the active fragment thereof used in the therapeutic methods of the present invention may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution. In example adding dipeptides can improve the penetration of a circulating agent in the eye through the blood retinal barrier by using endogenous transporters.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG) has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomular filtration (e.g., less than 45 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes. Such linkers may be used in modifying the protein or fragment of the protein described herein for therapeutic delivery.

In another preferred embodiment, the disease associated with retinal degenerative disorder may be a disease with retinal photoreceptors degeneration.

In another embodiment, retinal degenerative may have inherited toxic, metabolic causes or due to ageing.

### Nucleic acids, vectors, recombinant host cells and uses thereof

A second aspect of the invention relates to an isolated nucleic acid sequence coding for a protein according the claims 1 to 2 for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury.

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

These nucleic acid molecules may be obtained by conventional methods well known to those skilled in the art, in particular by site-directed mutagenesis of the gene encoding the native protein. Typically, said nucleic acid is a DNA or RNA molecule, which may be included in a suitable vector, such as a plasmid, cosmid, episome, artificial chromosome, phage or viral vector.

So, a further object of the present invention relates to a vector and an expression cassette in which a nucleic acid molecule of the invention is associated with suitable elements for controlling transcription (in particular promoter, enhancer and, optionally, terminator) and, optionally translation, and also the recombinant vectors into which a nucleic acid molecule in accordance with the invention is inserted. These recombinant vectors may, for example, be cloning vectors, or expression vectors.

The terms "vector", "cloning vector" and "expression vector" mean the vehicle by which a DNA or RNA sequence (e.g. a foreign gene) may be introduced into a host cell, so as to transform the host and promote expression (e.g. transcription and translation) of the introduced sequence.

Any expression vector for animal cell may be used, as long as a gene encoding a polypeptide or chimeric derivative of the invention can be inserted and expressed. Examples of suitable vectors include pAGE107, pAGE103, pHSG274, pKCR, pSG1 beta d2-4) and the like.

Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or 30 viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

Examples of promoters and enhancers used in the expression vector for animal cell include early promoter and enhancer of SV40, LTR promoter and enhancer of Moloney mouse leukemia virus, promoter and enhancer of immunoglobulin H chain and the like, and promoter specific of RPE or Muller cells.

Also disclosed are gene delivery systems comprising a nucleic acid molecule of the invention, which can be used in gene therapy in vivo or ex vivo. This includes for instance viral transfer vectors such as those derived from retrovirus, adenovirus, adeno associated virus, lentivirus, which are conventionally used in gene therapy. This also includes gene delivery systems comprising a nucleic acid molecule of the invention and a non-viral gene delivery vehicle. Examples of non viral gene delivery vehicles include liposomes and polymers such as polyethylenimines, cyclodextrins, histidine/lysine (HK) polymers, etc. also disclosed is a prokaryotic or eukaryotic host cell genetically transformed with at least one nucleic acid molecule according to the invention.

The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

Preferably, for expressing and producing the proteins, and in particular the human Transferrin, eukaryotic cells, in particular mammalian cells, and more particularly human cells, will be chosen.

Typically, cell lines such as CHO, BHK-21, COS-7, C127, PER.C6 or HEK293 could be used, for their ability to process to the right post-translational modifications of the derivatives.

The construction of expression vectors and the transformation of the host cells can be carried out using conventional molecular biology techniques. The V-ATPase c-subunit derivatives of the invention, can, for example, be obtained by culturing genetically transformed cells in accordance with the invention and recovering the derivative expressed by said cell, from the culture. They may then, if necessary, be purified by conventional procedures, known in themselves to those skilled in the art, for example by fractionated precipitation, in particular ammonium sulphate precipitation, electrophoresis, gel filtration, affinity chromatography, etc.

In particular, conventional methods for preparing and purifying recombinant proteins may be used for producing the proteins for use in accordance with the invention.

### Pharmaceutical compositions

A third object of this invention is 6. A pharmaceutical composition for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury comprising a therapeutically effective amount of human transferrin or an active fragment thereof according to the invention, or an nucleic acid according to to the invention, or a plasmid according to the invention, or an expression vector according to the invention along with at least one pharmaceutically acceptable excipient.

Said vector may be used in gene therapy.

By a "therapeutically effective amount" is meant a sufficient amount of the chimeric derivative of the invention to treat a disease associated with retinal degenerative disorder at a reasonable benefit/risk ratio applicable to any medical treatment.

It will be understood that the total daily dosage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The active products for use of the invention (proteins or vectors) may be administered for the treatment of a disease associated with retinal degenerative disorder.

The therapeutically effective amount of the active product [proteins or vectors (constructions)] that should be administered, as well as the dosage for the treatment of a pathological condition with the proteins and/or pharmaceutical compositions will depend on numerous factors, including the age and condition of the patient, the severity of the disturbance or disorder, the method and frequency of administration and the particular peptide to be used.

The presentation of the pharmaceutical compositions that contain the proteins or vectors (constructions) may be in any form that is suitable for administration, e.g., solid, liquid or semi-solid, such as creams, ointments, gels or solutions, and these compositions may be administered by any suitable means, for example, orally, parenterally, inhalation or topically, so they will include the pharmaceutically acceptable excipients necessary to make up the desired form of administration. A review of the different pharmaceutical forms for administering medicines and of the excipients necessary for obtaining same may be found, for example, in the "Tratado de Farmacia Gal nica" (Treatise on Galenic Pharmacy), C. Faul i Trillo, 1993, Luz n 5, S.A. Ediciones, Madrid.

In the pharmaceutical compositions for oral, sublingual, subcutaneous, intramuscular, intravenous, transdermal, local, pulmonary, eye drop, intraocular or rectal administration, the active principle, alone or in combination with another active principle, can be administered in a unit administration form, as a mixture with conventional pharmaceutical supports, to animals and human beings. Suitable unit administration forms comprise oral route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms, intraocular and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds for use according to the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The hTf or the active fragment thereof for use according to the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

For intraocular administration, the composition according to the invention may be by electroporated for example through a device described in the patent application WO2006123248, WO03030989.

The hTf or the active fragment thereof for use according to the invention may be formulated as a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration; liposomal formulations; time release capsules; and any other form currently used.

The invention will be further illustrated by the following examples. However, these examples should not be interpreted in any way as limiting the scope of the present invention.

### EXAMPLE:

### Example 1: Experimental results on Rd10 mice.

### Material & Methods

### PIXE experiments.

### Animal specimens

Rd10 mice and C57B1/6J control mice 2- to 4-week-old were fed with a standard laboratory diet and tap water ad libitum and maintained on a half light/half dark photoperiod in a temperature-controlled room at 21-23°C. All experimental procedures were performed in accordance with the Association for Research in Vision and Ophthalmology (ARVO) Statement for the Use of Animals in Ophthalmic and Vision Research.

Mice were sacrificed by carbon dioxide inhalation or by an overdose of sodium pentobarbital (Sanofi Santé Animale, Libourne, France). Eyes were enucleated immediately after euthanasia, mounted in Tissue-Tek (O.C.T) (Bayer Diagnostics, Puteaux, France), snap frozen with dry ice, and stored at -80°C until use.

### Sample preparation

Cryo-sections of 20 µm thickness were obtained with a cryomicrotome (Leica, Rueil-Malmaison, France). Sagittal sections close to the optic nerve were collected on aluminum holders coated previously with a Formvar film, then samples were freeze-dried overnight at-330°C, and finally stored in a dessicator over silica gel until analysis. A precise observation of the samples was made with an optical microscope (Carl Zeiss, Le Pecq, France) to choose the best preserved and best located zones for analysis.

### Analysis

The samples were analyzed with a nuclear microprobe in Bordeaux-Gradignan. The conditions were exactly the same as our previous studies. A 2.5 MeV proton beam of 800-900 pA and 5 µm diameter was used. The scanned areas were 150x150 µm2. X-rays were detected using a 80 mm2 Si(Li) solid state detector (Link analytical, Gif sur Yvette, France) and backscattered protons were detected with a 20 mm2 Si surface barrier detector placed at 135° to the beam direction. The organic mass of the analyzed samples and their thickness were calculated from the backscattered spectra using the RUMPIN code. Iron (Fe), copper (Cu), zinc (Zn) and potassium (K) concentrations were calculated from the X-ray intensities with the GUPIX software. Special attention was paid to the elemental losses induced by proton beam irradiation of the tissue.

After irradiation, the sections were stained with 1% toluidine blue or DAPI (4',6 Diamidino-2-phenyl-indole, Sigma, Saint Quentin Fallavier, France) diluted 1/2000, and observed with a microscope Aristoplan (Leica) equipped for fluorescence. The identification of the retinal layers in the adjacent zones allowed us to recognize them in the irradiated areas. For each layer, it was possible to get the PIXE and Rutherford Back Scattering spectra and then to determine the element concentrations. Data were expressed in µg/g dry weight tissue ± standard error.

### Rd10/hTf mice generation.

Rd10/hTf mice were generated from rd10 mice (βPDE-/-) and transgenic mice carrying the human transferrin gene (TghTf) both in the same genetic background (C57B1/6J). TghTf mice carriesying the long hTf gene (80 kb) comprising its long 5' - and 3' - regulatory sequences and its own promotor. Briefly, rd10 mice were crossbred with TghTf mice (hTf+) to produce heterozygous rd10/hTfmice (βPDE+/-/hTf+). ELISA assay permitted to screen for the presence hTf in the blood but not the number of copy. Heterozygous rd10/hTf mice were then crossbred with rd10 mice and only generated βPDE-/-/hTf+ mice were selected. βPDE-/-/hTf+ mice were then crossbred together for 5 subsequent generations. βPDE-/-/hTf+ mice were named rd10/hTf. To control that the crossbreeding had not affected on the process of retinal degeneration observed in rd10 mice, we also crossbred rd10 mice with Wild Type (WT) C57B1/6J mice. Generated mice, named rd10/WT, had the βPDE mutation but did not express hTf. To check the absence or presence of the mutated βPDE gene, PCR of DNA was performed from tail biopsies. DNA was extracted using a DNeasy Blood and Tissues kit column (Qiagen, Courtaboeuf, France).

### PCR.

The corresponding PCR amplification was performed in 35 cycles by denaturation at 95°C for 15 min; annealing at 94, 55, and 72°C, respectively, for 30 sec, 30 sec, and 2 min, and elongation at 72°C for 10 min. The product obtained was purified and digested with the enzyme HhaI (Invitrogen, Cergy Pontoise, France), whose restriction site is not included in the rd10 mutant DNA. After 1 hour incubation with the enzyme at 37°C, the digested DNA was run on an agarose gel for separation of short DNA fragments. The homozygous rd10 mutation was revealed by the presence of bands having a size of 1257 and 715 bp; the heterozygous mutation by bands having a size of 1257, 715, 661 and 54 bp; and none mutation by bands having a size of 1257, 661 and 54 bp.

### Human transferrin injection.

Five-day-old rd10 mice were i.p injected daily during 20 days, with Ca2+-free physiological buffer [Buffer Saline Sodium (BSS)] or with human apotransferrin solution (Sigma) at concentrations of 6, 12, 24, 48 mg/ml in BSS. During the first 7 days, mice received 0.1ml of hTf solution. The following 7 days, they received 0.2 ml and after up to the date of sacrifice, the volume of injection was of 0.3ml. Mice were sacrificed at 25 or 47 days post-natal for histological analysis and hTf quantification in neural retina and immunohistochemistry studies.

### Human transferrin quantification.

Mice were perfused through the left cardiac ventricle with 1X PBS. Five mg of neural retina were incubated in lysis buffer [15 mM Tris, pH 7.9, 60 mM KCl, 15 mM NaCl, 2 mM EDTA, 0.4 mM phenylmethylsulphonyl fluoride (PMSF) (Perbio Science, Brebiers, France)]. After four freeze/thaw cycles, lysates were centrifuged at 5000g for 10 min and supernatants were stored at -20°C. hTf was quantified by an antibody-sandwich ELISA using, as previously described, two different polyclonal antibodies directed against hTf : sheep anti-hTf (Biodesign, distributed by Invitrogen, Cergy Pontoise, France) and rabbit anti-hTf (F. Guillou, INRA, Tours-Nouzilly, France). The minimum limit for hTf detection was 0.1ng/ml. The cross-reaction rate for hTf antibodies with mouse Tf was less than 0.05%. All standards and samples were assayed in triplicate. Results were reported in ng/ml of supernatant.

### Histology.

Enucleated eyes from mice were fixed with 4% paraformaldehyde, 0.5% glutaraldehyde (LADD, Inland Europe), in PBS for 2 hours. After fixation, samples were washed, dehydrated and transferred into the infiltration solution of the Leica Historesin embedding kit (Leica) overnight at 4°C. Samples were embedded in the embedding medium (Leica) and attached to a block holder after polymerization. Plastic sections (5 µm thick) were prepared on a microtome (Leica), stick on slides coated with gelatin and stained 2 min with 1% Toluidin Blue solution. Sections were observed on a Aristoplan microscope (Leica) and photographed with a camera (Leica).

### Measurement of nuclear layers thickness.

For comparison of retinal morphology from 3-week-old rd10, rd10/WT and rd10/hTf mice, or between 25-day-old rd10 mice injected with BSS or hTf, the outer nuclear layer (ONL) and inner nuclear layer (INL) thickness were measured in sagittal sections made every 200 µm on both sides of the optic nerve (inferior and superior pole) and across the whole retina. For each protocol, the ONL measurements were made with Visilog 6.2 (Noesis) in 3 different sections close to the optic nerve from 2 to 6 eyes, and averaged out to a single value.

### Statistical analysis.

Statistical analysis was conducted using the Student's t-test and a P value < 0.05 was considered statistically significant. Measurements of retinal nuclear layers thickness from various groups were compared with two-way ANOVA test. Analysis was performed using GraphPad Prism 4 software. Each experimental condition was repeated three to four times on 3-6 samples each time. All results are presented as mean ± standard error of the mean (SEM).

### Immunohistofluorescence analysis.

Freshly enucleated eyes were fixed for 2 hours with 4% paraformaldehyde in 1X phosphate-buffered saline (PBS, Gibco distributed by Invitrogen, Cergy Pontoise, France), washed with PBS, mounted in Tissue-Tek O.C.T. (Siemens Medical, Puteaux, France) and frozen with dry ice. Frozen sections (10 µm) were cut on a microtome (Leica). Sections were incubated for 1 hour with different primary antibodies diluted in PBS: rabbit polyclonal anti-mTf, 1:100 (F. Guillou, INRA, France), rabbit polyclonal anti-hTf, 1:100 (F. Guillou, INRA, France), anti-rhodopsine (Rho4D2, generous gift from Dr. Molday), 1:200. Negative control sections were incubated with rabbit non-immune serum (Gibco) or without primary antibody. After washing with PBS, sections were incubated for 1 hour with secondary antibodies (goat anti-rabbit), labelled with Texas Red (Jackson Laboratories, distributed by TebuBio, Le Perray en Yvelines, France) or Alexa 488 (Molecular Probes, distributed by Invitrogen, Cergy Pontoise, France), both diluted 1:200 in PBS. Cones were directly labeled with peanut agglutinin conjugated with fluorescein isothiocyanate (Sigma). Nuclei were counterstained with DAPI. Labeled sections were observed under an epifluorescence microscope (Olympus, Rungis, France) and photographed with an Olympus camera (Olympus) using identical exposure parameters across samples to be compared.

### TUNEL labelling.

Terminal deoxynucleotidyl transferase-mediated biotinylated UTP nick end labeling (TUNEL) reaction was also carried out along with DAPI staining. The protocol was adapted from manufacturer's protocol (Roche Diagnostics, Meylan, France). Briefly, frozen retinal sections, obtained as described above, were fixed for 10 min with PFA, washed with PBS 1X, and incubated for 2 min with 0.1% Triton X-100 in 0.1% sodium citrate on ice. Then, sections were incubated for 60 min at 37°C with the reaction mixture (TUNEL enzyme plus TUNEL label (1/9)) and finally, nuclei were counterstained with DAPI.

### Results

### Iron distribution in Rd10 mice retina by PIXE analysis.

Iron, copper, zinc and potassium ion contents were analyzed by PIXE microanalysis in retina from rd10 mice and WT mice. Analysis was performed at 2, 3 and 4 weeks of age to capture early, peak and late stages of degeneration, respectively. Potassium content was the same in both rd10 and WT mice and did not change with age between two and four weeks. Total iron (heme and non heme) content analysis demonstrated a significant increase in the area corresponding to PR inner and outer segments in rd10 mice retina compared to WT mice at all ages studied. Retinal iron levels in rd10 mice compared with age-paired controls were increased between 1.71-, 2.45-, and 3.15-fold at 2-, 3- and 4-week-old respectively. Moreover, iron accumulation in the outer neural retina of rd10 mice was significantly increased in a time-dependent manner (by 23% between 2 and 3 weeks and by 51% between 3 and 4 weeks), whereas in WT mice, it remained at the same level. Zinc and copper content were also significantly higher in the same area in rd10 mice compared with same-age controls. In rd10 mice retina, zinc increase was age-dependent, whereas copper content was statistically significant higher only between 3 and 4 weeks. Thus by PIXE analysis, we demonstrated that iron, but also zinc and copper content were higher in rd10 mice compared to WT mice.

### Rd10/hTf mice analysis

### hTf expression

We generated rd10/hTf and rd10/WT mice by crossbreeding rd10 mice with TghTf mice or WT mice. Genotyping has permitted to discriminate the presence of βPDE mutation. We quantified in neural retina, the concentration of hTf in 3-week-old rd10, rd10/WT and rd10/hTf mice compared to TghTf mice. As expected, there was no hTf expression in rd10 and rd10/WT mice eyes. In neural retina, hTf concentration in rd10/hTf mice was similar to that in TghTf mice. These results were confirmed by immunodetection of hTf in retinal sections of the 4 types of mice. Immunohistochemisty for hTf in retina from rd10 and rd10/WT mice were negative and similar to the control incubated with no immune serum. hTf was localized in the same layers in rd10/hTf and TghTf mice retina, i.e. astrocytes, MGC, INL, outer and inner plexiform layers, inner segments and RPE. Immunodetection of Cellular Retinaldehyde Binding Protein (CRALBP), a specific marker for MGC, allowed observing that hTf was located in MGC bodies within the inner nuclear layer and in MG cell processes that form radial extensions through the retina.

### Retinal degeneration

We have considered retinal degeneration at the peak of degeneration for rd10 mice, i.e. in 3-week-old rd10, rd10/WT and rd10/hTf mice. We analyzed PR loss and inner retina modifications by measurement of ONL (Outer nuclear layer) and INL (Inner nuclear layer) thickness, respectively. As expected, no differences were found in ONL thickness between rd10 and rd10/WT mice. However, the ONL thickness was significantly more preserved in rd10/hTf mice than in rd10 or rd10/WT mice. This difference was only statistically significant in the inferior pole, but not in the superior pole. From 400 µm to 2000 µm in the inferior pole, ONL thickness in rd10/hTf mice was 2.6-fold higher than in the two controls rd10 and rd10/WT mice. Average of ONL thickness in the superior and inferior poles in 3-week-old TghTf mice was 43.9 µm. In rd10/hTf mice, average ONL thickness in the superior and inferior poles were 59% and 36% lower (p<0.001 superior pole; p<0.05 inferior pole) respectively in comparison with TghTf.

In the INL at the superior pole, there was no significant difference between rd10, rd10/WT and rd10/hTf mice. However, INL thickness at the inferior pole from rd10/hTf, was 1.3 and 1.5 higher than in rd10 mice and rd10/WT mice, respectively. Not significant differences were found between the INL thickness in the two poles in rd10/hTf and TghTf mice (data not shown).

We also detected the apoptosis in PR nuclei by TUNEL method in 3-week-old rd10, rd10/WT, rd10/hTf and TghTf mice. In the medial portion of the inferior pole, many PR nuclei in apoptosis in rd10 and rd10/WT mice were observed, whereas only few TUNEL positive PR nuclei were detected in rd10/hTf mice and none in TghTf mice retinas.

The distribution and the morphology of cones and rods were analyzed by immunohistofluorescence in retinal cross-sections of all types of studied mice. Rd10 and rd10/WT mice cones labeled with peanut agglutinin, seemed to be sparse, globular with collapsed outer segments (OS), thin, small and stunted. However, the cones from rd10/hTf mice shown to be more elongated and numerous than in rd10 mice, and similar to those of TghTf mice. In the other hand, rods immunodetected with Rho4D2 antibody showed a similar pattern in both rd10 and rd10/WT mice. The thickness of rods OS was more preserved in rd10/hTf mice than in the 2 rd10 mice. hTf expression in rd10/hTf mice seemed to protect the 2 types of PR, but more especially rods.

### Human transferrin injection in Rd10 mice.

### hTf retinal content

We tested the potential protective role of hTf on retinal degeneration, by daily intraperitoneal injection with 4 different concentrations (6, 12, 24 and 48 mg/ml) of hTf in 5-day-old rd10 mice. We determined if after i.p. injection, hTf could cross the blood-retinal barrier and reside within the retina. The concentration of hTf was quantified by ELISA in neural retinal lysates 20 days after the beginning of daily injections, which corresponds roughly to 3 weeks post-natal and to the peak of major retinal degeneration in rd10. A dose dependent effect between the quantity of hTf injected and the local hTf concentration was found in the retina, with a plateau reached with 24 mg/ml.

### Retinal degeneration

The neuroprotective capacity of hTf, was determined measuring the ONL and the INL thickness each 200µm on the totality of superior and inferior poles in retina of rd10 mice injected with vehicle (BSS) or hTf solutions at 24 and 48mg/ml. As we observed in the retinal sections from 25-day-old rd10 mice treated with BSS and stained with toluidin blue, the ONL completely disappeared, and only one to two rows of PR nuclei remained closed to the RPE layer. The treatment with 24 mg/ml and in major degree 48 mg/ml of hTf rescued, the overall structure, i.e. the length of the OS as well as the density of rod nuclei (or the ONL thickness). A precise analysis of nuclear layer thickness in animals treated with 24 mg/ml of hTf, illustrated that the ONL was preserved in the major part of the retina, but statistically more significantly between 400 and 800 µm at the superior pole (on average: 2.5-fold higher in mice injected with 24mg/ml hTf than BSS) and between 800 and 2000 µm at the inferior pole (on average: 2-fold higher in mice injected with 24 mg/ml hTf than BSS). In rd10 mice injected with 48mg/ml of hTf, the ONL thickness was significantly higher between 6.3- and 3.7-fold at the superior and inferior poles, respectively, than in rd10 mice injected with BSS. The ONL thickness of rd10 mice injected with 48 mg/ml of hTf was not statistically different of the ONL in TghTf mice in the 2 poles (data not shown).

Analysis of the INL revealed that the superior pole shows a same pattern of retinal thickness in rd10 mice injected with BSS or with 24mg/ml of hTf, but it was much higher in the inferior pole. By contrast, the INL thickness of rd10 mice injected with 48 mg/ml of hTf showed a pattern thicker in both poles than mice injected with BSS. There was no difference between mean INL thickness of rd10 mice injected with 48 mg/ml hTf and TghTf mice.

These results were confirmed by PR apoptosis detection in retinal sections of rd10 mice injected with BSS or hTf evaluated by TUNEL reaction. We detected many PR nuclei in apoptosis in the ONL of rd10 mice injected with BSS, whereas in hTf injected rd10 mice, only few apoptotic (TUNEL positive) nuclei were found. Injection of hTf had a protective effect on retinal degeneration, by reducing the loss of PR by apoptosis.

Finally, we determined the hTf effects on PR cell rescue in rd10 mutant mice injected with BSS or with two different hTf doses (24 and 48 mg/ml). We observed that cones from rd10 mice injected with BSS were round and disorganized similar to the non-injected rd10 mice. However, in rd10 animals injected with hTf, as in rd10/hTf or TghTf mice, the cones were elongated and more numerous than in rd10 mice injected with BSS. On the other hand, rods in rd10 mice injected with BSS also shown pathological characteristics of the rod degeneration similar to those rods from rd10 or rd10/WT animals. But, in those rd10 mice injected with hTf, the number and the length of rods were clearly higher than those mice injected with BSS. The effect was more pronounced in rd10 mice injected with a dose of 48 mg/ml than in mice injected with 24mg/ml of hTf. In rd10 mice treated with 24 mg/ml of hTf, rods labelling was similar to that of rods in rd10/hTf mice retina. The rods layer from rd10 mice injected with 48mg/ml of hTf was characteristically similar to TghTf mice. Thus, high doses of hTf showed a strong protective effect on rod and cones outer segments integrity.

### DISCUSSION

Iron is an essential component of cell survival, however its capacity to generate highly reactive hydroxyl free radicals via the Fenton reaction can result in toxicity for the cells. Iron overload is found in human retina during aging (Sergeant C, personal communication; Hahn P et al., Neuroreport, 2006) and has been associated with retinal degeneration in AMD patients. Moreover, iron homeostasis defects in aceruloplasminemia patient (Dunaief JL et al., Ophthalmology, 2005) or in Cp-/-Heph-/Y mice are directly associated with retinal degeneration. Two rodent models of retinal degeneration caused by an inherited mutation have been demonstrated to present an iron retinal excess and a modification of iron regulating proteins expression: RCS rats with a mutation in Mertk gene and rd10 mice with a mutation in βPDE gene (Yefimova MG et al., IOVS, 2002; Deleon E et al., IOVS, 2009). The rd10 mutant is a phenotype characterized by a slowly progressing retinal degeneration, which has had the potential to become a model to study the cell biology of this disease and to test therapeutic tools. Results from the present study using PIXE analysis, show an alteration in iron metabolism during retinal degeneration in rd10 mice. These results were similar to the previously reported in animals evaluated at 3-week-old. We detected an increase of total (heme and non heme) iron content in inner and outer segments of PR from rd10 mice compared to control mice and it accumulation was age-dependant. The major part of total iron content may be bound in ferritin complex since Deleon E et al. found an increased ferritinbound iron in rd10 mice retina. Also, our results showed that more than 2/3 of iron content was localized in the inner segments of PR (Sergeant C, personal communication), containing metabolic machinery of PR and mitochondria, which needed iron available. Zinc levels also showed an age-dependent increment in rd10 animal, whereas copper content was only increased between 3 and 4 weeks postpartum. Zinc, like iron, is found in sub-RPE deposit formation in AMD patients and it is increased in RCS rat. Human disorders in copper metabolism such as Menkes disease and Wilson disease can result in retinal degeneration.

Our results show that the progressive degeneration of PR leads to increased release of (bound or unbound) iron (also zinc and copper) that promote itself PR death. An altered metabolism of iron, zinc and copper concentrations may play an important role in oxidative stress associated with the time course of retinal degeneration progression.

Furthermore, we investigated the potential protective effect of hTf in the rd10 model of retinal degeneration. We previously demonstrated, in vitro, that hTf expressed by MGC from TghTf mice in culture or added to the culture medium of MGC from WT mice, had the same protective role against oxidative stress induced by iron excess (Picard E, et al., 2008). Our study, based on these two in vitro methods was applied in vivo in rd10 mice: one by generating rd10 mice expressing hTf and other one by injection of hTf directly in rd10 mice.

For the first strategy, we generated a mouse homozygous for βPDE mutation and hTf gene: rd10/hTf mice. This mouse expressed the same hTf content in retina than TghTf mice. Moreover, the crossbreeding did not modify the hTf transgene construct because the protein was localized like in human retina and TghTf mice retina. hTf was expressed in the whole retina and especially in RPE and MGC (Picard E, et al., 2008). The ONL thickness was more preserved and TUNEL positive cells were less numerous in rd10/hTf mice than in rd10 mice. In addition, hTf preserved partly the number of rods and the morphology of cones. Thus, hTf expression in rd10/hTf, at the level of TghTf mice retina, rescued PR lost due to the βPDE mutation. PR death induced structural changes in neuronal postsynaptic cells which resulted in the INL thickness decrease. In rd10/hTf mice, there was a higher INL thickness than in rd10 mice, demonstrating the hTf neuroprotective role not only on PR cones and rods but also for the preservation of others neurons. The percentage of hTf protection was higher in inferior pole than in superior pole, which supposed a gradient-like hTf expression in rd10/hTf mice retina.

For the second strategy, we injected every day 24 and 48 mg/ml of apohTf (iron free) in 5-day-old rd10 mice before the onset of retinal degeneration. We chose the i.p. route instead of the intravitreal (i.v.) one to avoid the risk ocular infection and variation in intraocular pressure. We noted that the immunostaining of hTf in retina of mice injected with hTf (data not shown) was diffused but stronger in choriod and in retinal capillaries. Hunt and Davis (1992, J cell Physiol) demonstrated that Tf from choriocapillaries can be bound by RPE on basal membrane and release to other side. Human transferrin haves 50% of homology with mouse transferrin proteins so we can suppose that hTf can be bound by mouse transferrin receptor. Burdo JR et al. (2003, Neurosciences) demonstrated that the way of iron delivery throughout the inner blood-retina barrier could be Tf receptor-mediated transcytosis. The protective effect and hTf concentration in retina was proportional to the concentration of hTf injected. The effect of hTf injection was measured 20 days after the beginning of the protocol, at 25-day-old, the peak of retinal degeneration. When 24 and 48 mg/ml were injected, the hTf concentration in retina was nearly the same than in rd10/hTf and TghTf mice. However, the retina of rd10 mice injected with 48 mg/ml of hTf had kept almost all PR compared to rd10 mice injected with BSS. The ONL thickness was nearly the same than in TghTf mice or WT mice. The retina of these animals showed only few TUNEL positive PR and cones and rods morphology were was intact. Also, the INL thickness was similar at the INL in TghTf mice, demonstrating the preservation of secondary neurons. But, in rd10 mice injected with 24mg/ml, the PR rescue was less substantial than in 48 mg/ml hTf injected mice. Nevertheless, the rescue of PR was significant compared with mice injected with BSS. The difference in the ONL thickness rescue and the hTf concentration in retina of rd10 mice injected with 24 and 48mg/ml, could be explained by a quantity limited of Tf that could be present in retina. The rest of hTf remained available in central circulation for the retina and a regulatory mechanism may exist to regulate Tf entrance in retina (Garcia-Castineiras S et al., 2010, Exp Eye Res).

The discrepancy between the almost equal level of hTf in retina of rd10/hTf mice or mice injected with hTf and the different level of PR protection could be explained by the limited capacity of retinal cells (in which RPE and MGC) to produce hTf in rd10/hTf mice while the blood concentration of hTf in mice injected was elevated, available and could be regulated.

The two strategies permitted hTf presence in retina of mice and reduced retinal degeneration. In rd10 mice retina, PR death produced an oxidative stress and a release of increasing iron content. Deleon et al. demonstrated that Tf, ceruloplasmin, ferritin, Tf receptor were increased in 3-week-old rd10 mice retina. So in response to iron excess, rd10 retina increase Tf expression to manage and transport iron. In your study, we experimentally increase Tf level. but at a higher level than in normal rd10, to access an amount sufficient to chelate and decrease iron released and possessed iron in excess. We propose that hTf may bound iron released during PR degeneration to decrease the iron toxic level and thus, to decrease oxidative stress. hTf has already been studied for its potential as anti-oxidant. In renal ischemia-reperfusion injury, Tf lowered the circulating redox-active iron levels. Tf has potent anti-apoptotic/cytoprotective effects against Fas-mediated signals in hepatocytes and lymphohaemopoietic cells. In a rabbit model of after-cataract formation following cataract surgery, Tf synthesis is upregulated in lens epithelia acting as a survival and proliferative factor. Here, we noted that in rd10/hTf mice and in rd10 mice injected with hTf, mouse Tf is increased in retina, especially in outer segment and ganglion cell layer (data not shown). This data confirmed your previous study where we demonstrated that hTf expression in TgTfH transgenic mice increase mouse Tf expression, and enhanced a possible loop of autoregulation control for transferrin expression similar in the two mouse species (Picard E et a., 2008, Mol Vis).

The present study shows that hTf, an endogenous iron-binding protein, preserved rods and cones which have been demonstrated to be highly sensitive to iron excess. hTf protected also secondary neurons and thus preserved possibly neuronal post-synaptic connections. Other iron homeostasis proteins, like heavy chain of ferritin can also participate in the control of iron excess as we have shown recently in aging or in light induced stress retina of heavy chain of ferritin heterozygous mice (Picard et al., 2010, under review). These results show that one of the major central actors in many neuronal degenerative diseases in the eye, as in the brain, is the ability of free iron to generate free radicals and be a major part of oxidative stress mechanism. How long such a treatment needs to be pursued in order to protect cones on the long term remains to be determined but it is already known that Tf is not toxic by itself at a high dose and administrated during a long period. Preliminary experiences demonstrated the limiting role of hTf to protect retina over 47 days post-natal (data not shown). Is this oxidative stress mechanism also involved in the protection of interneurons (amacrin, bipolar and horizontal cells) between PN0 and PN15 from the programmed cell death during development of the mouse retina? How neurotrophic actors, like CNTF or FGF interact with these iron dependeant oxidative mechanisms is under investigation? Tf has also multiple biological functions not related to its iron binding capacity but to other properties such as its ability to bond insulin-like growth factor binding protein 3. With the use of a biological agent like hTf, we could override the potential use of pharmaceutical chelators, such as deferoxamine which were toxic at high doses or other chelators which have been proposed to protect retina. In this study, we found a correlation of iron homeostasis imbalance with neurodegenerative state of the retina in rd10 mice and show for the first time, a protective role of hTf in an animal model accumulating iron in PR. This therapeutic strategy could be applicable to others degenerative models. We proposed that the defect in phagocytosis disturbed iron recycling and resulted in Tf degradation. We previously demonstrated that in RCS rat, there is an accumulation of iron closed to area of degenerative PR and a decrease of Tf expression (Yefimova MG et al., IOVS, 2002). Thus, injection of hTf in RCS rat may provide rescue of PR. Moreover, hTf injection could be envisaged in AMD, where increase chelatable iron was observed in RPE and Bruch's membrane. AREDS (ArchOpthalmol, 2001) study haves already demonstrated the beneficial effect of a diet enriched with anti-oxidant nutriments on the development of AMD.

Intraperitoneal administration is of particular interest since it highlights the therapeutically potential of Tf to protect iron-induced PR death that occurred in degenerative ocular diseases.

### Example 2: Effects of human transferrin injected in the vitreous of rats against the damages induced by exposure to intense illumination.

### Material & Methods

7 weeks old Wistar rats were preinjected with different amounts of human transferrin in the vitreous under anesthesia. 5 µl of hTf at 50mg/ml (SIGMA ref T5391)or a diluted solution were injected in the eye. The animal were kept in the dark for 6h and then put in individual cage under intense green led (3500lux) for 12 hours.

After this period they were kept for 7 days in 12h hours dark and light cycle.

The integrity of their retina was observed by a special OCT technique designed for rat ocular inspection. The animals were then sacrificed, their eyes dissected and their retina processed as usual to determine the organization and the thickness of the outer nuclear layers (ONL) under microscopic examination.

### Results

The data obtained on both eyes of each animal demonstrated that the rats injected with the control solution BSS had lost in the superior part of their retina the majority of photoreceptors nuclear layers. Only one or less nuclear layer of sparse nuclei remained on more than 2/3 of the sections starting from the optic nerve.

In the eyes injected with the more concentrated solution of hTF in these same regions more than half the original thickness (5-9 nuclei) layer was conserved. In the eyes injected with this solution diluted 5 times a protection of one third of the original thickness (11 nuclei layer) was maintained. These data were confirmed by the OCT analysis.

These results demonstrated that the intraocular injection of hTf prior the illumination protects the eyes of Wistar rats against the degeneration of the photoreceptors in a dose dependant way.

This is an additional model of retinal degeneration in which we demonstrate for the first time that a treatment by hTf is neuroprotectiv. This model is the most widely used in most pharmacological assays of a potential neuroprotective effect of a compound. The technology used in these experiments is validated by that used currently with antiangiogenic drugs in human.

### REFERENCES:

Throughout this application, various references describe the state of the art to which this invention pertains.
AREDS. Risk factors associated with age-related macular degeneration. A case-control study in the age-related eye disease study: (2000) Age-Related Eye Disease Study Report Number 3. Ophthalmology. 07:2224-32.
Barhoum R, Martinez-Navarrete G, Corrochano S, Germain F, Fernandez-Sanchez L, de la Rosa EJ, de la Villa P, Cuenca N (2008) Functional and structural modifications during retinal degeneration in the rd10 mouse. Neuroscience 155:698-713.
Burdo, J.R., Antonetti, D.A., Wolpert, E.B., Connor, J.R., (2003) Mechanisms and regulation of transferrin and iron transport in a model bloodebrain barrier system. Neuroscience 121, 883-890.
Cassia R, Besnard L, Fiette L, Espinosa de los Monteros A, Ave P, Py MC, Huerre M, de Vellis J, Zakin MM, Guillou F (1997) Transferrin is an early marker of hepatic differentiation, and its expression correlates with the postnatal development of oligodendrocytes in mice. J Neurosci Res 50:421-432.
Chang B, Hawes NL, Pardue MT, German AM, Hurd RE, Davisson MT, Nusinowitz S, Rengarajan K, Boyd AP, Sidney SS, Phillips MJ, Stewart RE, Chaudhury R, Nickerson JM, Heckenlively JR, Boatright JH (2007) Two mouse retinal degenerations caused by missense mutations in the beta-subunit of rod cGMP phosphodiesterase gene. Vision Res 47:624-633.
Davidson MG, Harned J, Grimes AM, Duncan G, Wormstone IM, McGahan MC (1998) Transferrin in after-cataract and as a survival factor for lens epithelium. Exp Eye Res 66:207-215.
Deleon E, Lederman M, Berenstein E, Meir T, Chevion M, Chowers I (2009) Alteration in iron metabolism during retinal degeneration in rd10 mouse. Invest Ophthalmol Vis Sci 50:1360-1365.
De Vries B, Walter SJ, von Bonsdorff L, Wolfs TG, van Heurn LW, Parkkinen J, Buurman WA (2004) Reduction of circulating redox-active iron by apotransferrin protects against renal ischemia-reperfusion injury. Transplantation 77:669-675.
De Vries B, Walter SJ, von Bonsdorff L, Wolfs TG, van Heurn LW, Parkkinen J, Buurman WA (2004) Reduction of circulating redox-active iron by apotransferrin protects against renal ischemia-reperfusion injury. Transplantation 77:669-675.
Dingle J, Havener WH (1978) Ophthalmoscopic changes in a patient with Wilson's disease during long-term penicillamine therapy. Ann Ophthalmol 10:1227-1230.
Dunaief JL, Richa C, Franks EP, Schultze RL, Aleman TS, Schenck JF, Zimmerman EA, Brooks DG (2005) Macular degeneration in a patient with aceruloplasminemia, a disease associated with retinal iron overload. Ophthalmology 112:1062-1065.
Eckhert CD (1981) Elevated body zinc in rats with inherited retinal dystrophy. J Hered 72:130.
Ellingsen DG et al., (2007) Copper. In: Fowler, B.A., Norberg, M., Friberg, L., Norberg, G. (Eds.), Handbook of Toxicology of Metals. Academic Press, Inc.;
García-Castiñeiras S. (2010) Iron, the retina and the lens: A focused review. Experimental Eye Research 90 664-678
Hahn P, Ying GS, Beard J, Dunaief JL (2006) Iron levels in human retina: sex difference and increase with age. Neuroreport 17:1803-1806.
Hunt, R.C., Davis, A.A., (1992). Release of iron by human retinal pigment epithelial cells. J. Cell. Physiol. 152, 102-110.
Komeima K, Rogers BS, Campochiaro PA (2007) Antioxidants slow photoreceptor cell death in mouse models of retinitis pigmentosa. J Cell Physiol 213:809-815.
Lengyel I, Flinn JM, Peto T, Linkous DH, Cano K, Bird AC, Lanzirotti A, Frederickson CJ, van Kuijk FJ (2007) High concentration of zinc in sub-retinal pigment epithelial deposits. Exp Eye Res 84:772-780.
Lesnikov V, Lesnikova M, Deeg HJ (2001) Pro-apoptotic and anti-apoptotic effects of transferrin and transferrin-derived glycans on hematopoietic cells and lymphocytes. Exp Hematol 29:477-489.
Lesnikov VA, Lesnikova MP, Shulman HM, Wilson HM, Hockenbery DM, Kocher M, Pierpaoli W, Deeg HJ (2004) Prevention of Fas-mediated hepatic failure by transferrin. Lab Invest 84:342-352.
Lukinova N., Iacovelli J., Dentchev T., Wolkow N., Hunter A., Amado D., Ying G. S., Sparrow J. R. and Dunaief J. L. (2009).Iron chelation protects the retinal pigment epithelial cell line ARPE-19 against cell death triggered by diverse stimuli. Invest Ophthalmol Vis Sci 50:1440-7.
Picard E, Fontaine I, Jonet L, Guillou F, Behar-Cohen F, Courtois Y, Jeanny JC (2008) The protective role of transferrin in Muller glial cells after iron-induced toxicity. Mol Vis 14:928-941.
Rogers B. S., Symons R. C., Komeima K., Shen J., Xiao W., Swaim M. E., Gong Y. Y., Kachi S. and Campochiaro P. A. (2007).Differential sensitivity of cones to iron-mediated oxidative damage. Invest Ophthalmol Vis Sci 48:438-45.
Saleh MC, Espinosa de los Monteros A, de Arriba Zerpa GA, Fontaine I, Piaud O, Djordjijevic D, Baroukh N, Garcia Otin AL, Ortiz E, Lewis S, Fiette L, Santambrogio P, Belzung C, Connor JR, de Vellis J, Pasquini JM, Zakin MM, Baron B, Guillou F (2003) Myelination and motor coordination are increased in transferrin transgenic mice. J Neurosci Res 72:587-594.
Sergeant C, Gouget B, Llabador Y, Simonoff M, Yefimova M, Courtois Y, Jeanny J.C (1999) Nuclear Instruments and Methods in Physics Research 158: 344-348.
Ugarte M, Osborne NN (2001) Zinc in the retina. Prog Neurobiol 64:219-249.
Waggoner DJ, Bartnikas TB, Gitlin JD (1999) The role of copper in neurodegenerative disease. Neurobiol Dis 6:221-230.
Yefimova MG, Jeanny JC, Keller N, Sergeant C, Guillonneau X, Beaumont C, Courtois Y (2002) Impaired retinal iron homeostasis associated with defective phagocytosis in Royal College of Surgeons rats. Invest Ophthalmol Vis Sci 43:537-545.

## Claims

1. Human Transferrin or an active fragment thereof for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury.

2. The human transferrin or an active fragment thereof for use according to claim 1 wherein the disease is age-related macular degeneration.

3. An isolated nucleic acid sequence coding for a protein according the claims 1 to 2 for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury.

4. A plasmid comprising a nucleic acid sequence according to claim 3 for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury.

5. An expression vector containing a nucleic acid sequence according to claim 3 for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury.

6. A pharmaceutical composition for use in the treatment of a disease associated with retinal degenerative disorder selected from the group consisting of retinitis Pigmentosa, age-related macular degeneration, aceruloplasminemia, Bardet-Biedel syndrome, Bassen-Kornzweig syndrome, Best disease, choroidema, gyrate atrophy, Leber congenital amaurosis, Refsum disease, Stargardt disease, cataract, glaucomatous neuropathy and retinopathy of prematury comprising a therapeutically effective amount of human transferrin or an active fragment thereof according to claims 1 or 2, or an nucleic acid according to claim 3, or a plasmid according to claim 4, or an expression vector according to claim 5 along with at least one pharmaceutically acceptable excipient.

7. A pharmaceutical composition for use according to the claim 6 comprising a therapeutically effective amount of human transferrin.

## Patentansprüche

1. Human-Transferrin oder ein wirksames Fragment davon zur Verwendung bei der Behandlung einer Krankheit, verbunden mit degenerativer retinaler Störung, ausgewählt aus der Gruppe, bestehend aus Retinitis Pigmentosa, altersbedingter Makuladegeneration, Aceruloplasminämie, Bardet-Biedel-Syndrom, Bassen-Kornzweig-Syndrom, Best-Krankheit, Choroideremie, Atrophia gyrata, Leberscher kongenitaler Amaurose, Refsum-Syndrom, Stargardt-Krankheit, Katarakt, glaukomatöser Neuropathie und Frühgeborenen-Retinopathie.

2. Human-Transferrin oder ein wirksames Fragment davon zur Verwendung nach Anspruch 1, wobei die Krankheit altersbedingte Makuladegeneration ist.

3. Isolierte Nukleinsäure-Sequenz, die für ein Protein nach einem der Ansprüche 1 bis 2 kodiert, zur Verwendung bei der Behandlung einer Krankheit, verbunden mit degenerativer retinaler Störung, ausgewählt aus der Gruppe, bestehend aus Retinitis Pigmentosa, altersbedingter Makuladegeneration, Aceruloplasminämie, Bardet-Biedel-Syndrom, Bassen-Kornzweig-Syndrom, Best-Krankheit, Choroideremie, Atrophia gyrata, Leberscher kongenitaler Amaurose, Refsum-Syndrom, Stargardt-Krankheit, Katarakt, glaukomatöser Neuropathie und Frühgeborenen-Retinopathie.

4. Plasmid, umfassend eine Nukleinsäure-Sequenz, nach Anspruch 3 zur Verwendung bei der Behandlung einer Krankheit, verbunden mit degenerativer retinaler Störung, ausgewählt aus der Gruppe, bestehend aus Retinitis Pigmentosa, altersbedingter Makuladegeneration, Aceruloplasminämie, Bardet-Biedel-Syndrom, Bassen-Kornzweig-Syndrom, Best-Krankheit, Choroideremie, Atrophia gyrata, Leberscher kongenitaler Amaurose, Refsum-Syndrom, Stargardt-Krankheit, Katarakt, glaukomatöser Neuropathie und Frühgeborenen-Retinopathie.

5. Expressionsvektor, enthaltend eine Nukleinsäure-Sequenz nach Anspruch 3 zur Verwendung bei der Behandlung einer Krankheit, verbunden mit degenerativer retinaler Störung, ausgewählt aus der Gruppe, bestehend aus Retinitis Pigmentosa, altersbedingter Makuladegeneration, Aceruloplasminämie, Bardet-Biedel-Syndrom, Bassen-Kornzweig-Syndrom, Best-Krankheit, Choroideremie, Atrophia gyrata, Leberscher kongenitaler Amaurose, Refsum-Syndrom, Stargardt-Krankheit, Katarakt, glaukomatöser Neuropathie und Frühgeborenen-Retinopathie.

6. Pharmazeutische Zusammensetzung zur Verwendung bei der Behandlung einer Krankheit, verbunden mit degenerativer retinaler Störung, ausgewählt aus der Gruppe, bestehend aus Retinitis, Pigmentosa, altersbedingter Makuladegeneration, Aceruloplasminämie, Bardet-Biedel-Syndrom, Bassen-Kornzweig-Syndrom, Best-Krankheit, Choroideremie, Atrophia gyrata, Leberscher kongenitaler Amaurose, Refsum-Syndrom, Stargardt-Krankheit, Katarakt, glaukomatöser Neuropathie und Frühgeborenen-Retinopathie, umfassend eine therapeutisch wirksame Menge von Human-Transferrin oder einem wirksamen Fragment davon nach Ansprüchen 1 oder 2, oder einer Nukleinsäure nach Anspruch 3 oder einem Plasmid nach Anspruch 4 oder einem Expressionsvektor nach Anspruch 5 zusammen mit mindestens einem pharmazeutisch verträglichen Exzipienten.

7. Pharmazeutische Zusammensetzung zur Verwendung nach Anspruch 6, umfassend eine therapeutisch wirksame Menge von Human-Transferrin.

## Revendications

1. Transferrine humaine ou un fragment actif de celle-ci destinée à être utilisée dans le traitement d'une maladie associée à une maladie dégénérative de la rétine choisie dans le groupe constitué par la rétinite pigmentaire, la dégénérescence maculaire liée à l'âge, l'acéruléoplasminémie, le syndrome de Bardet-Biedl, le syndrome de Bassen-Kornzweig, la maladie de Best, la choroïdérémie, l'atrophie gyrée, l'amaurose congénitale de Leber, la maladie de Refsum, la maladie de Stargardt, la cataracte, la neuropathie glaucomateuse et la rétinopathie des prématurés.

2. Transferrine humaine ou un fragment actif de celle-ci destinée à être utilisée selon la revendication 1, où la maladie est la dégénérescence maculaire liée à l'âge.

3. Séquence d'acide nucléique isolée codant pour une protéine selon les revendications 1 à 2, destinée à être utilisée dans le traitement d'une maladie associée à une maladie dégénérative de la rétine choisie dans le groupe constitué par la rétinite pigmentaire, la dégénérescence maculaire liée à l'âge, l'acéruléoplasminémie, le syndrome de Bardet-Biedl, le syndrome de Bassen-Kornzweig, la maladie de Best, la choroïdérémie, l'atrophie gyrée, l'amaurose congénitale de Leber, la maladie de Refsum, la maladie de Stargardt, la cataracte, la neuropathie glaucomateuse et la rétinopathie des prématurés.

4. Plasmide comprenant une séquence d'acide nucléique selon la revendication 3, destiné à être utilisé dans le traitement d'une maladie associée à une maladie dégénérative de la rétine choisie dans le groupe constitué par la rétinite pigmentaire, la dégénérescence maculaire liée à l'âge, l'acéruléoplasminémie, le syndrome de Bardet-Biedl, le syndrome de Bassen-Kornzweig, la maladie de Best, la choroïdérémie, l'atrophie gyrée, l'amaurose congénitale de Leber, la maladie de Refsum, la maladie de Stargardt, la cataracte, la neuropathie glaucomateuse et la rétinopathie des prématurés.

5. Vecteur d'expression comprenant une séquence d'acide nucléique selon la revendication 3, destiné à être utilisé dans le traitement d'une maladie associée à une maladie dégénérative de la rétine choisie dans le groupe constitué par la rétinite pigmentaire, la dégénérescence maculaire liée à l'âge, l'acéruléoplasminémie, le syndrome de Bardet-Biedl, le syndrome de Bassen-Kornzweig, la maladie de Best, la choroïdérémie, l'atrophie gyrée, l'amaurose congénitale de Leber, la maladie de Refsum, la maladie de Stargardt, la cataracte, la neuropathie glaucomateuse et la rétinopathie des prématurés.

6. Composition pharmaceutique destinée à être utilisée dans le traitement d'une maladie associée à une maladie dégénérative de la rétine choisie dans le groupe constitué par la rétinite pigmentaire, la dégénérescence maculaire liée à l'âge, l'acéruléoplasminémie, le syndrome de Bardet-Biedl, le syndrome de Bassen-Kornzweig, la maladie de Best, la choroïdérémie, l'atrophie gyrée, l'amaurose congénitale de Leber, la maladie de Refsum, la maladie de Stargardt, la cataracte, la neuropathie glaucomateuse et la rétinopathie des prématurés, comprenant une quantité thérapeutiquement efficace de transferrine humaine ou d'un fragment actif de celle-ci selon la revendication 1 ou 2, ou un acide nucléique selon la revendication 3, ou un plasmide selon la revendication 4, ou vecteur d'expression selon la revendication 5 avec au moins un excipient pharmaceutiquement acceptable.

7. Composition pharmaceutique destinée à être utilisée selon la revendication 6, comprenant une quantité thérapeutiquement efficace en transferrine humaine.
